# EUROPEAN PATENT SPECIFICATION

(11) **EP 2 907 484 B1**
(45) Date of publication and mention of the grant of the patent: **08.08.2018**
(21) Application number: 15155126.4
(22) Date of filing: 13.02.2015
(51) Int. Cl.: A61F 2/78, A61F 2/50, A61F 2/30

(54) **Percutaneous osseointegrated implant assembly for use in supporting an exo-prosthesis**
Perkutane knochenintegrierte Implantatanordnung zur Verwendung bei der Unterstützung einer Exoprothese
Ensemble à implant osséo-intégré percutané destiné à être utilisé pour supporter une exo-prosthèse

(30) Priority: 14.02.2014 US 201414181029
(43) Date of publication of application: 19.08.2015
(73) Proprietor: University of Utah Research Foundation, Salt Lake City, UT 84108 (US); Longo, Joseph A., Paradise Valley, AZ 85253 (US)
(72) Inventor: Longo, Joseph A., Paradise Valley, Arizona 85253 (US); Bachus, Kent N., Salt Lake City, Utah 84117 (US); Jeyapalina, Sujee, Salt Lake City, Utah 84013 (US); Beck, James Peter, Salt Lake City, Utah 84193 (US); Bloebaum, Roy, Salt Lake City, Utah 84184 (US); Agarwal, Jayant P., Salt Lake City, Utah 84103 (US); Kubiak, Erik, Salt Lake City, Utah 84105 (US); Holt, Brian Mueller, Topanga, California 90290 (US)
(74) Representative: EIP

(56) References cited:
- WO-A1-2013/048589
- WO-A1-2014/015303
- US-A1- 2003 109 878
- US-A1- 2007 060 891

## Description

This invention generally relates to implant assemblies that are adapted to be securely attached to a long bone and to support an exo-prosthesis. More particularly, the invention relates to such assemblies that area adapted for attachment to a long bone by osseointegration.

Amputation of limbs can occur as a result of trauma or surgical intervention. Currently, despite its limitations, socket technology remains the standard of care for attachment and/or docking of exo-prosthetic devices to a residual amputated limb of a patient. Recent clinical reviews of amputees with socket prosthetics have suggested that between 8% and 50% of amputees with socket prosthetics suffer from one or more dermatological pathologies that require temporary suspension of use of a prosthetic. The inability of these amputees to consistently use their prosthetics represents a significant decrease in their quality of life. In lower limb amputees, the limitations on usage of a prosthetic increase the susceptibility of the amputees to additional co-morbidities. Many pathologies related to current socket prosthetic designs possess interconnected biochemical and biomechanical cues.

Known implant assemblies are described in various published patent applications, including: WO2013/048589 (University of Utah Research Foundation et al, 4 April 2013), upon which the preamble of appended claim 1 is based; WO2014/015303 (University of Utah Research Foundation et al, 23 January 2014); US2007/060891 (Skiera Richard et al, 15 March 2007); and US2003/109878 (Grundei Hans, 12 June 2003).

High infection rates remain a major limitation of current prosthetic systems. A high infection rate is often associated with the lack of a skin seal at the skin- implant interface that provides an ideal direct path for opportunistic bacterial invasion to the stomal tissue and often results in sinus tract formation. This may subsequently result in deep infection and bone loss requiring implant removal. Because of the rapid evolution of antibiotic-resistant pathogens as well as the high incidence of methicillin- resistant *Staphylococcus aureus* (MRSA) cases, these infections may not be treatable with conventional antibiotic therapy. Commonly, the outcome is device removal and further loss of bone and soft tissue resulting in shortening of the residual limb.

Therefore, what is needed in the art is an inherently adaptable, modular percutaneous osseointegrated prosthetic implant assembly that permits formation of a seal at the implant-skin interface, reduces dermatological complications associated with socket prosthetics, improves proprioception, extends the periods during which an exoprosthesis can be worn, and reduces energy expenditure of the amputee wearing the prosthetic, thereby improving the overall quality of life for the amputee. In accordance with the present invention there is provided an implant assembly as defined in independent claim 1. Preferred embodiments are defined in the dependent claims. Exemplary methods in which an implant assembly according to the present invention may be used are also disclosed in the following description.

Among the advantages of the present invention is that it provides an implant assembly that can be readily installed in a long bone such as the femur. This assembly allows for the formation of a skin seal at the implant-skin interface. Thus, it is anticipated that this assembly will reduce dermatological complications associated with socket prosthetics and will improve proprioception. Furthermore, it is expected that this implant assembly will extend the period during which an exo-prosthesis can be worn, and will reduce the ambulation energy expenditure of the amputee wearing the prosthetic, thereby improving his/her overall quality of life.

Other advantages and features of this invention will become apparent from an examination of the drawings and the ensuing description.

The use of the terms "a", "an", "the" and similar terms in the context of describing the invention are to be construed to cover both the singular and the plural, unless otherwise indicated herein or clearly contradicted by context. The word "or" as used herein means any one member of a particular list and also includes any combination of members of that list. The terms "comprising", "having", "including" and "containing" are to be construed as open-ended terms (i.e., meaning "including, but not limited to,") unless otherwise noted. The terms "substantially", "generally" and other words of degree are relative modifiers intended to indicate permissible variation from the characteristic so modified. The use of such terms in describing a physical or functional characteristic of the invention is not intended to limit such characteristic to the absolute value which the term modifies, but rather to provide an approximation of the value of such physical or functional characteristic. The terms "optional", "optionally" and similar terms mean that the subsequently described event or circumstance may or may not occur, and that the description includes instances where said event or circumstance occurs and instances where it does not.

The steps of all methods described herein can be performed in any suitable order unless otherwise specified herein or clearly indicated by context. The use of any and all examples or exemplary language (e.g., "such as") herein is intended merely to better illuminate the invention and not to place a limitation on the scope of the invention, unless otherwise indicated by the claims. Nothing in the specification should be construed as indicating any non-claimed element as essential to the practice of the invention. Ranges can be expressed herein as from "about" one particular value to "about" another particular value. Such an expression is intended to include a range from the one particular value to the other particular value, as well as ranges including variations in the particular values.

Several terms are specifically defined herein. These terms are to be given their broadest possible construction consistent with such definitions, as follows:

The term "subject" refers to an individual, and can include humans as well as other animals. The term "subject" does not denote a particular age or sex, and can include animals of either sex and of any age.

The term "insertional" refers to the end of a first component that is configured for insertion into a second component. For example, an "insertional" end of a component can be configured for insertion into a prepared site within a long bone or for insertion into an implant component.

The term "low energy surface" refers to a surface comprising or coated with one or more materials that are configured to inhibit adhesion and/or adsorption between the surface and other materials. For example, "low energy surfaces" as described herein permit little if any in-growth, integration, and/or adhesion between the surface and biological tissues, biological fluids, and bacteria. It is contemplated that the reduced bio-adhesion permitted by the "low energy surfaces" described herein can permit thorough post-operative drainage for improved wound healing and reduce the incidence of infection. Exemplary low energy surfaces include surfaces that comprise or are coated with hydrophobic materials (i.e., those having a 100°-150° contact angle) or superhydrophobic materials (i.e., those having >150° contact angle).

The term "soft tissue" refers to all or part of a subject's epidermis, dermis and underlying subcutaneous tissues.

The invention comprises an implant assembly for a long bone, which assembly is adapted to support an exo-prosthesis. The assembly includes a stem having a proximal end and a distal end, a subdermal component that is adapted for attachment to the distal end of the stem, and a percutaneous post. The proximal end of the stem is adapted to be received in a surgically prepared medullary canal of the long bone, and the distal end has a surface that is adapted to promote bone ingrowth. The subdermal component includes a proximal portion and a fixation surface. The proximal portion of the subdermal component is adapted for attachment to the distal end of the stem. At least one of the distal end of the stem and the proximal portion of the subdermal component defines a surface that is adapted to engage an osteotomy created by a cut through the long bone that is generally perpendicular to the longitudinal axis of the long bone. The percutaneous post has a proximal end that is adapted to be attached to the subdermal component. Preferably, it is the proximal portion of the subdermal component that defines the surface that is adapted to engage an osteotomy. The stem may include a plurality of splines and/or a central slot. The implant assembly may include a stoma shield that is adapted to be located adjacent a distal end of the subdermal component.

In order to facilitate an understanding of the invention, the preferred embodiments of the invention and the best mode known by the inventors for carrying out the invention are illustrated in the drawings, and a detailed description thereof, as well as a description of presently contemplated alternatives, follows. It is not intended, however, that the invention be limited to the particular embodiments described or to use as specifically described herein. Therefore, the scope of the invention contemplated by the inventors includes all equivalents of the subject matter described herein, as well as various modifications and alternative embodiments such as would ordinarily occur to one skilled in the art to which the invention relates. The inventors expect skilled artisans to employ such variations as seem to them appropriate, including the practice of the invention otherwise than as specifically described herein. In addition, any combination of the elements, components and steps of the invention described herein in any possible variation is encompassed by the invention, unless otherwise indicated herein or clearly excluded by context.

The presently preferred embodiments of the invention are illustrated in the accompanying drawings, in which like reference numerals represent like parts throughout, and in which:
Figure 1 is a side view of a first embodiment of the invention.
Figure 2 is a side of the embodiment shown in Figure 1, showing the addition of a stoma shield.
Figure 3 is a sectional view of the embodiment shown in Figure 2.
Figure 4 is a partially exploded view of the embodiment of Figures 2 and 3.
Figure 5 is a side view of a second embodiment of the stem coupled to a second embodiment of the subdermal component.
Figure 6 is a sectional view of the embodiment shown in Figure 5.
Figure 7 is a side view of the stem and the subdermal component of the embodiment of Figures 1-4.
Figure 8 is a side view of a third example of the stem coupled to a third example of the subdermal component which is not part of the present invention.
Figure 9 is a side view of the stem of the example of Figure 8 coupled to a fourth example of the subdermal component which is not part of the present invention.
Figure 10 is a side view of the embodiment of the percutaneous post that is illustrated in Figures 1-4.
Figure 11 is a side view of the percutaneous post of Figure 10, which view is rotated 90° about the long axis of the post from that shown in Figure 10.
Figure 12 is a front view of the stoma shield that is illustrated in Figures 2 and 3.
Figure 13 is a side view of the stoma shield of Figure 12.
Figure 14 is a side view of adapter that is illustrated in Figures 1-4.
Figure 15 is a side view of the assembly bolt that is illustrated in Figure 4.
Figure 16 is a side view of the adapter bolt that is illustrated in Figure 4.

The angles and dimensions depicted in the drawings may be exaggerated for clarity and, consequently, may not be to scale.

The following description of the invention is provided as an enabling teaching of the invention in its best, currently known embodiments. To this end, those skilled in the relevant art will recognize and appreciate that many changes can be made to the various aspects of the invention described herein, while still obtaining the beneficial results of the present invention. It will also be apparent that some of the desired benefits of the present invention can be obtained by selecting some of the features of the present invention without utilizing other features. Accordingly, those who work in the art will recognize that many modifications and adaptations to the present invention are possible and can even be desirable in certain circumstances and are a part of the present invention. Thus, the following description is provided as illustrative of the principles of the present invention and not in limitation thereof.

Disclosed herein are implants, implant assemblies, and methods for securing an exo-prosthesis to a limb of a subject. It is contemplated that the disclosed implants, implant assemblies, and methods can be used to permit integration of bone and skin of the subject into an implant to which an exo-prosthesis is operably attached. It is further contemplated that the disclosed implants, implant assemblies, and methods can permit formation of a seal between the soft tissue of the subject and the implant, thereby minimizing the possibility of peri-prosthetic infections, both superficial soft tissue and muscle infections and deep bony infections. It is still further contemplated that the disclosed implants, implant assemblies, and methods can minimize post-implantation migration of the soft tissue of the subject, thereby reducing the likelihood of formation of a pocket between the skin of the subject and the implant. In particular applications, it is contemplated that the disclosed implants, implant assemblies, and methods can be used to allow military veterans and civilians who have experienced lower extremity amputation to a obtain a near pre-amputation activity level. Generally, it is contemplated that the above-mentioned objectives can be achieved through optimized selection of various characteristics of the implant assembly, such as, for example, surface characteristics of the various components described herein, to achieve a desired arrangement and/or orientation of the implant assembly to the bone and surrounding tissue of the subject, as well as a desired attachment between the implant and an exo-prosthesis.

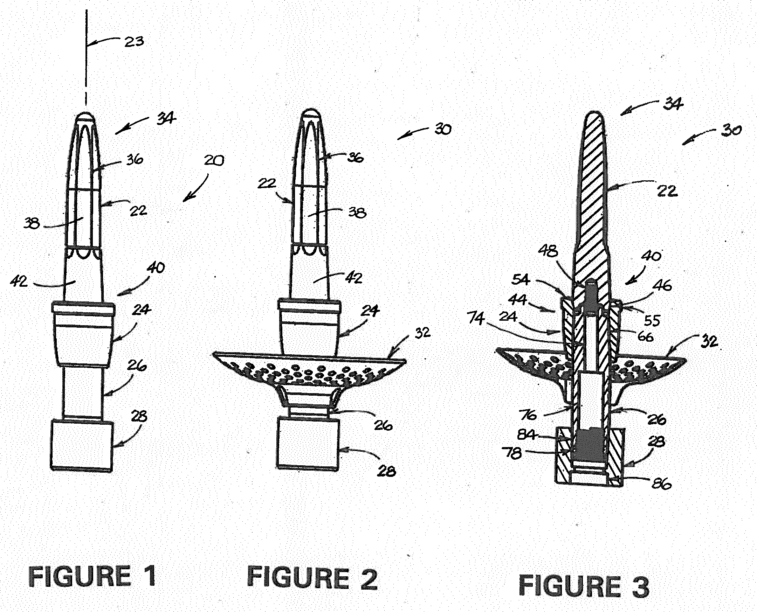

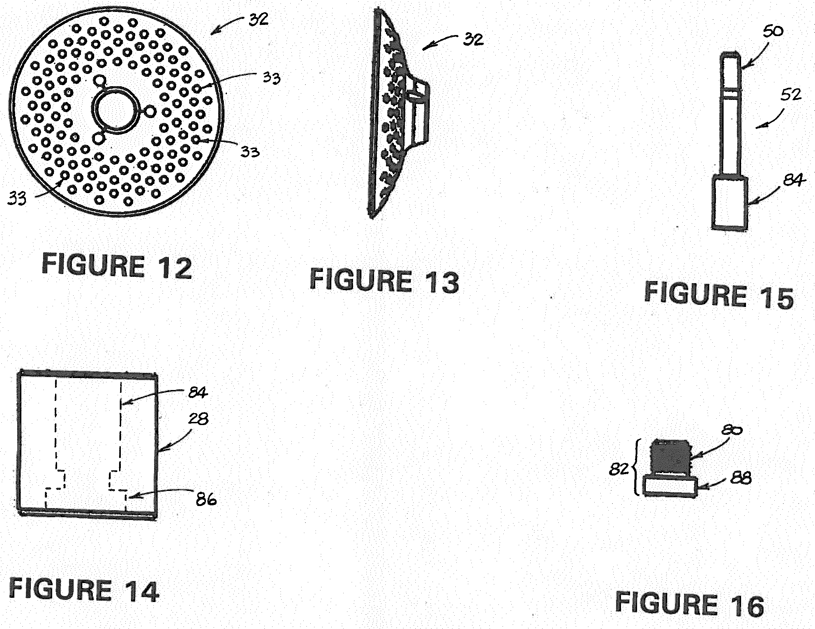

As shown in Figure 1, implant assembly 20 includes stem 22, subdermal component 24, percutaneous post 26 and adapter 28. Each of these components can be made of conventional surgical-quality metallic materials, including, for example and without limitation, titanium, cobalt chrome, and the like. Figures 2-4 illustrate implant assembly 30, which is essentially identical to implant assembly 20 except for the addition of stoma shield 32 (also illustrated in Figures 12 and 13). The stoma shield is preferably comprised of a flexible elastomeric material and includes a plurality of ventilation holes 33. It is adapted to fit over and provide a measure of protection for the amputation stump of a subject, as well as a means for protecting the exo-prosthetic from natural body fluid that may weep from the stoma during healing.

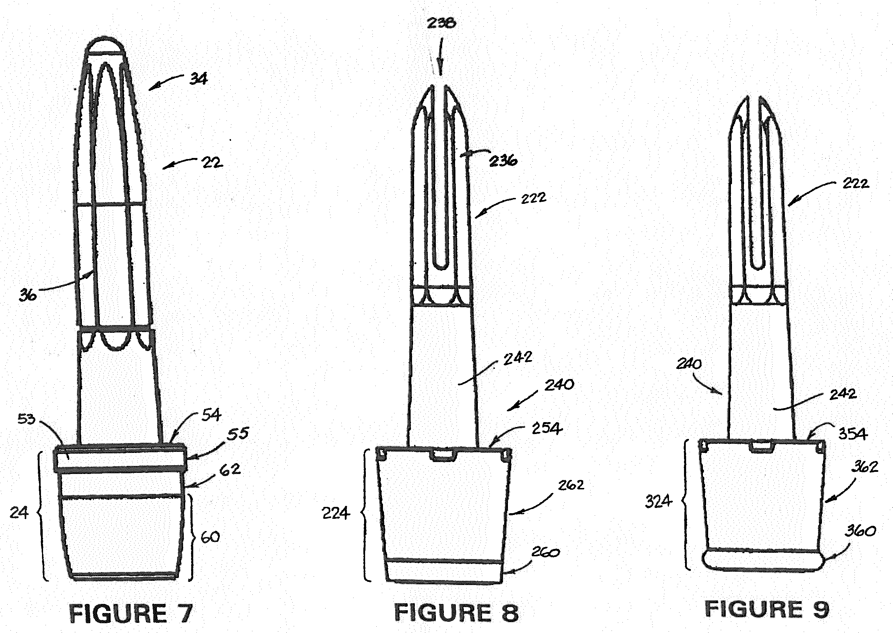

Stem 22, which is also illustrated in Figure 7, has a proximal (or insertional) end 34 that is adapted to be received in a prepared hole that has been bored in the intramedullary cavity of a long bone such as the femur of a subject whose lower leg has been amputated. However, it is also contemplated that the disclosed methods, assemblies and components thereof can be used with any long bone within a tissue region of a subject, including, for example, a humerus of a subject. It is also contemplated that stem 22 can be securely received within the prepared hole such that the longitudinal axis 23 of the stem is substantially axially aligned with the longitudinal axis of the selected bone. In the embodiment of stem 22 that is illustrated in the drawings, splines 36 are provided to enhance fixation within the bone. Preferably, the stem includes a plurality of spaced splines 36 that are oriented substantially parallel to the longitudinal axis 23 of the stem. It is also contemplated that at least a portion of the outer surface 38 of the insertional or proximal end 34 of the stem 22 can be treated to obtain a desired surface roughness, thereby improving bone attachment.

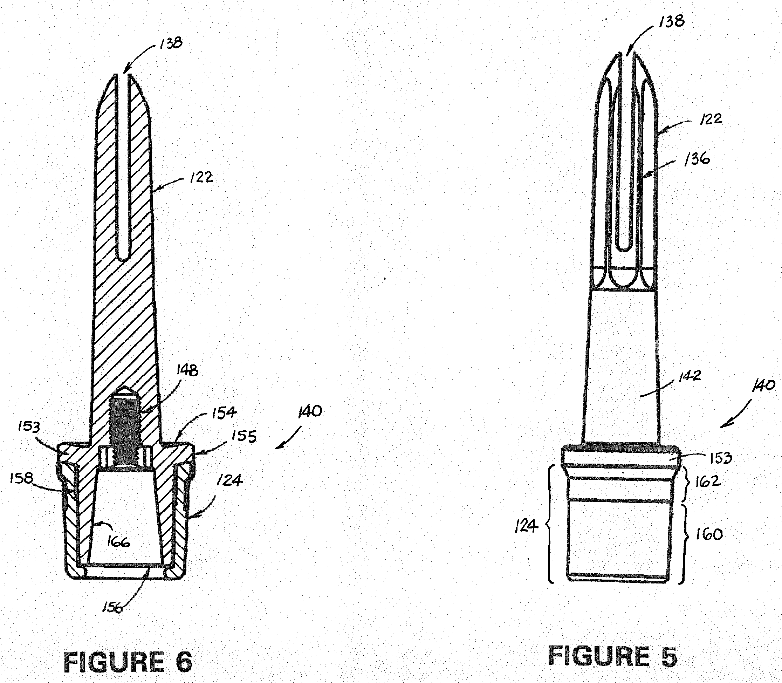

A second embodiment of the stem is illustrated in Figures 5 and 6, and a third example is illustrated in Figures 8 and 9. Stem 122 (shown in Figures 5 and 6) includes a plurality of longitudinal splines 136 and a slot 138 that is configured to promote fixation with the selected bone. Similarly, stem 222 (shown in Figures 8 and 9) includes a plurality of longitudinal splines 236 and a slot 238 that is configured to promote fixation with the selected bone. Optionally, stems 22, 122 and 222 can be configured to mimic physiological properties of the anatomical bow within a native bone, thereby reducing and/or limiting torsional displacement of the stem following implantation, and improving both short-term and long-term performance of the implant assembly.

Stem 22 is also provided with a distal end 40 having a surface 42 that is adapted to promote bone ingrowth. Stems 122 and 222 are each provided with similar distal ends 140 and 240 respectively and similar surfaces 142 and 242 respectively. Surface 42 preferably comprises a porous structure that is adapted to support osseointegration. This porous structure may be obtained by coating at least a portion of the distal end with a porous material using known methods for coating a substrate. Preferably, porous surface 42 comprises a continuous circumferential layer of porous material. Alternatively, porous surface 42 can comprise a plurality of porous sections that are spaced about an operative circumference of the distal end of the stem. Although not shown in the drawings, the porous surface 42 of the stem can be recessed relative to the outer surface of adjoining portions of the stem. It is contemplated that the porous surface 42 of the stem can be formed using known techniques. Preferably, porous surface 42 comprises porous titanium, and more preferably, substantially pure porous titanium. However, it is also contemplated that other medical grade porous metals, as well as porous polymers and ceramics, can be used as described herein. Preferably, the porous surface 42 of the stem can have a thickness ranging from about 0.5 mm to about 2.0 mm. It is also preferred that the porosity of the porous surface 42 of the stem can range from about 40% to about 80% and, more preferably, from about 50% to about 70%. It is also preferred that the size of each pore of the porous surface 42 can range from about 25 µm to about 1,000µm and, more preferably, from about 40 µm to about 400 µm. However, as one will appreciate, it is contemplated that the desired porosity and pore size for the porous surface 42 can be selectively varied depending upon, for example and without limitation, the subject's bone condition, amputation causality, the age of the subject, the residual limb length, the time since initial injury, the time since amputation, the vascular health of the subject, and other factors.

It is contemplated that, upon secure receipt of stem 22 within the surgically prepared site, the porous surface 42 of distal end 40 of the stem can be configured to promote integration and ingrowth of the selected bone of the subject into the stem. It is further contemplated that such osseointegration (between the selected bone and the porous surface 42 of the stem 22) can lead to improvement in the post-surgery quality of life of the subject through improved proprioception (i.e., dynamic exo-prosthesis load sensation) and gait efficiency (i.e., improved physiologic energy expenditure). It is also contemplated that the modular design of the implant assembly described herein can provide a platform for further optimization of the design of the stem without the need for significant surgical intervention. For example, it is contemplated that the porous region can be limited in size so as to permit removal and/or revision of the stem with minimal loss or destruction of residual bone. It is further contemplated that, in the event of a bone infection following implantation of the stem, the stem can be removed, the surgically revised site can be allowed to drain, and a therapeutic treatment regimen can be executed without significant tissue loss or harm to the subject.

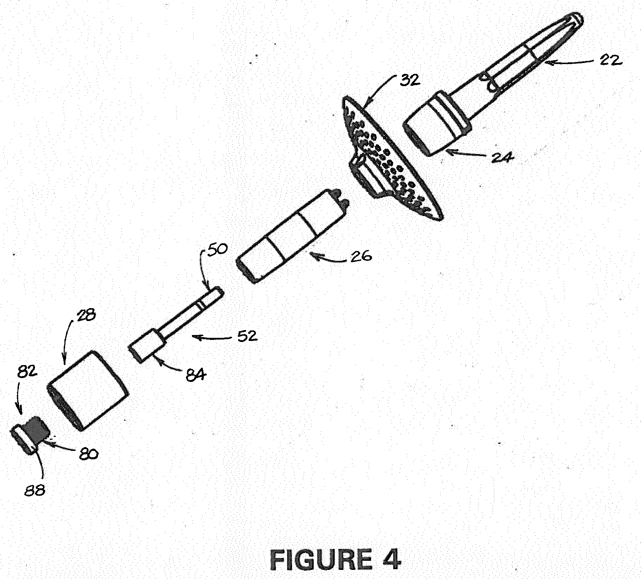

Assemblies 20 and 30 also include subdermal component 24 having a proximal end 44 that is adapted for attachment to the distal end of the stem. Preferably, the subdermal component is hollow. In the embodiment of the invention best illustrated in Figure 3, terminal portion 46 of the distal end 40 of stem 22 is tapered and adapted to be received in a corresponding tapered portion of the proximal end 44 of the subdermal component. In this embodiment of the invention, it is also preferred that distal end 40 of the stem includes threaded bore 48 and that proximal portion 50 of assembly bolt 52 (shown in Figures 4 and 15) is provided with corresponding male threads so that the assembly bolt may be attached by threaded engagement to the stem. In the embodiments of the invention illustrated in the drawings, at least one of the distal end of the stem and the proximal portion of the subdermal component defines a collar that extends radially outwardly from adjacent structures and is adapted to engage an osteotomy created by a cut through the long bone that is generally perpendicular to the longitudinal axis of the long bone. Thus, in the embodiment of the invention illustrated in Figures 1-4 and 7, subdermal component 24 includes a proximal end portion that defines a collar 53 which extends radially outwardly from the adjacent distal end 40 of the stem and is adapted to engage an osteotomy created by a cut through the long bone that is generally perpendicular to the longitudinal axis of the long bone. In the embodiment of the invention illustrated in Figures 5 and 6, the distal end 140 of the stem includes a proximal collar 153 that extends radially outwardly from adjacent portions of the stem and is adapted to engage an osteotomy created by a cut through the long bone that is generally perpendicular to the longitudinal axis of the long bone.

The proximal collar portion of either the stem or the subdermal component can be configured to form a flush interface and/or seal with the selected bone. It is further contemplated that the flush interface and/or seal formed between the collar and the cut bone can permit transfer of ground reaction forces substantially directly to the skeletal system of the subject, thereby preventing bone atrophy. Preferably, the proximal collar portion includes a substantially flat surface 54 or 154 that extends substantially perpendicularly relative to the longitudinal axis of the stem. However, it is contemplated that the proximal collar portion 53 or 153 can include a chamfered surface or have another surface shape, provided the collar surface permits formation of a flush interface with the cut in the bone. It is further contemplated that the proximal collar portion can be shaped to substantially conform to the shape of adjacent tissues within the subject. In one embodiment, the proximal collar portion can have a substantially circular cross-sectional profile about the longitudinal axis of the stem. However, it is also contemplated that the proximal collar portion can have an elliptical or other cross-sectional profile that permits formation of a flush interface with the selected bone and provides desired support to the selected bone. Optionally, the radially outer surface 55 or 155 of the proximal collar portion can be outwardly tapered, sloped, and/or curved with respect to the longitudinal axis of the stem so as to substantially conform to the shape of adjacent tissue structures within the subject. In addition, at least a portion of the surface of the collar that is adapted to abut bone can comprise a porous material as described herein in order to facilitate and increase bone integration, thereby ensuring contiguous load transmission between the implant assembly and the distal end of the bone. It is further contemplated that appropriately scaled load transmission at this interface can prevent bone resorption due to stress shielding at the distal bone end of the transected bone and can promote bone hypertrophy more proximally.

Distal end 140 of stem 122 also includes downwardly depending portion 156 below proximal collar portion 153. At least a portion 158 of the outer surface of this downwardly depending portion is provided with male threads that are adapted to mate with corresponding female threads on subdermal component 124, so that the subdermal component can be attached to the stem by threaded engagement as shown in Figure 6. The distal end 140 of stem 122 also includes threaded bore 148 (similar to threaded bore 48 in stem 22), so that proximal end 50 of assembly bolt 52 (shown in Figures 4 and 15), which is provided with corresponding male threads, may be attached by threaded engagement to the stem. Also contemplated within the scope of the invention are other mechanisms by which the subdermal component can be attached to the stem that are known to those having ordinary skill in the art to which the invention relates.

As indicated by comparing Figures 5-9, the configuration of the subdermal component may vary. In some embodiments of the subdermal component, the portion below the collar is substantially cylindrical. In other embodiments, the configuration of the subdermal component may be tapered or concave towards its distal end, or convex towards its distal end. In some embodiments of the invention, the proximal end of the subdermal component includes an abutment surface, such as abutment surface 254 of subdermal component 224 (shown in Figure 8) or abutment surface 354 of subdermal component 324 (shown in Figure 9), that permits formation of a flush interface with the cut in the bone but does not extend radially outwardly from adjacent portions of the subdermal component. In other embodiments, the proximal end portion of the subdermal component may have a larger dimension than adjacent portions.

The outer surfaces of the subdermal component are provided with one or more surface treatments to facilitate a secure attachment of the implant assembly to the long bone and to permit the formation of a seal between the soft tissue of the subject and the implant assembly, thereby minimizing the possibility of infection at or below the interface between the implant assembly and the soft tissue of the subject. In accordance with the present invention, the subdermal component is provided with an outer surface having discrete areas or portions with differing surface treatments, including a fixation surface, a low energy surface, and a transition surface between the fixation surface and the low energy surface. Thus, as best shown in Figure 7, distal portion 60 is provided with a low energy surface that is adapted to inhibit bio-adhesion, proximal collar portion 53 is provided with a fixation surface 55 that is adapted to promote soft tissue fixation, and intermediate portion 62 is provided with a transition surface between the fixation surface and the low energy surface. In similar fashion, distal portion 160 of subdermal component 124 (shown in Figure 5) is provided with a low energy surface that is adapted to inhibit bio-adhesion, proximal collar portion 153 is provided with a fixation surface 155 that is adapted to promote soft tissue fixation, and intermediate portion 162 is provided with a transition surface between the fixation surface and the low energy surface. In some examples of the subdermal components which are not part of the claimed invention, there is no portion having a transition surface. Thus, as shown in Figure 8, distal portion 260 of subdermal component 224 is provided with a fixation surface that is adapted to promote soft tissue fixation and proximal portion 262 is provided with a low energy surface that is adapted to inhibit bio-adhesion. Similarly, as shown in Figure 9, distal portion 360 of subdermal component 324 comprises a ring of larger diameter than the adjacent part of proximal portion 362, which ring is provided with a fixation surface that is adapted to promote soft tissue fixation. In this example of the invention, proximal portion 362 is provided with a low energy surface that is adapted to inhibit bio-adhesion.

The low energy surface of the distal portion of the subdermal component (such as distal portion 60 of subdermal component 24) comprises a surface that has been treated to increase its hydrophobic characteristics. Such a surface may be obtained by treating the surface by a process selected from the group consisting of abrasive blasting, burnishing, grinding, buffing, chemical vapor deposition and combinations of such processes. The fixation surface of the proximal portion of the subdermal component (such as fixation surface 55 of proximal collar portion 53 of subdermal component 24) is adapted to facilitate soft tissue integration. Such a surface may be obtained by treating the surface to produce or apply a porous structure such as a structure that is adapted to support osseointegration. The transition surface of the intermediate portion of the subdermal component (such as intermediate portion 62 of subdermal component 24) comprises a surface that has been treated to increase its surface energy relative to the low energy surface, yet still have a lower surface energy than the fixation surface. Such a surface may be obtained by treating the surface by a process selected from the group consisting of abrasive blasting, burnishing, grinding, buffing, chemical vapor deposition and combinations of such processes.

The inner surface of the subdermal component is preferably tapered to receive the correspondingly tapered proximal end portion 68 of percutaneous post 26. Thus, inner surface 66 of subdermal component 24 is preferably tapered to receive the proximal end portion of the percutaneous post, and the inner surface 166 of downwardly depending portion 156 of stem 122 is also preferably tapered to receive the proximal end portion of the percutaneous post. The taper of these surfaces, and that of other tapered surfaces described herein, can comprise conventional Morse tapers. Alternatively, such surfaces may be tapered relative to the longitudinal axis 23 of the stem 22 at an angle ranging from about 1.5° to about 4° and, more preferably, from about 2.5° to about 3.5°.

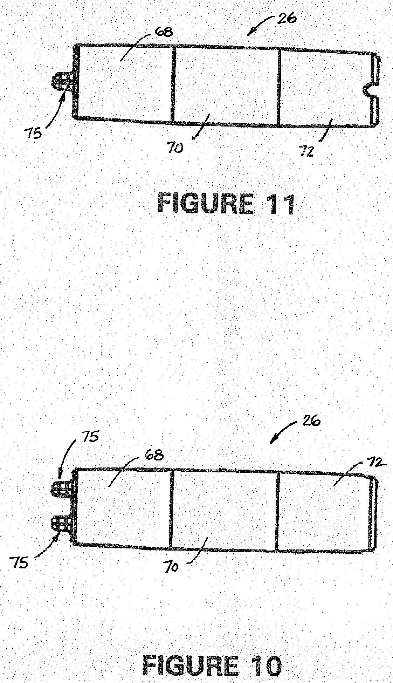

Percutaneous post 26 also includes a central portion 70 that is preferably of generally cylindrical configuration and a tapered distal portion 72. In the embodiments of the invention illustrated in the drawings, percutaneous post 26 is provided with a central bore, as shown in Figure 3. This central bore has a proximal portion 74 of a first diameter and a distal portion 76 of a second diameter. Th e proximal end 68 of percutaneous post 26 is also provided with one or more anti-rotation tabs 75, shown in Figures 10 and 11 (two are shown in the drawings) that allow for transmission of assembly torques through the tabs and the percutaneous post and away from the bone-implant interface.

The percutaneous post is provided with a low energy surface such as is provided on the distal portion of the subdermal component. Such surface may be obtained by treating it to reduce the likelihood of biological attachment, such as by treating the surface by a process selected from the group consisting of abrasive blasting, burnishing, grinding, buffing, chemical vapor deposition and combinations of such processes. The distal end 78 of distal portion 76 of the percutaneous post is threaded to receive threaded end 80 of adapter bolt 82.

The implant assembly also preferably includes an adapter 28 having a central bore which includes a first portion 84 and a second portion 86, as shown in Figure 3. The first portion 84 of the central bore is preferably tapered so as to receive distal portion 76 of the percutaneous post, whereas the second portion 86 of the central bore is shaped so as to receive head 88 of adapter bolt 82. A plurality of adapters may be included to provide functional interfaces with various exo-prostheses, or a single adapter may be provided for a plurality of exo-prostheses.

The implant assembly also preferably includes an assembly bolt 52 comprising a proximal portion 50 with a threaded proximal end that is adapted to engage threaded bore 48 in the distal end of the stem. Proximal portion 50 has a proximal diameter that is adapted to be received in bore 74 in the proximal portion of the central bore of percutaneous post 26 so that the threaded end of assembly bolt 52 may engage threaded bore 48 in the distal end of the stem. Assembly bolt 52 also has a distal portion 84 having a distal end and a distal diameter that is adapted to be received in distal portion 76 of the central bore of the percutaneous post, said distal end being provided with a hex-head, locking nut or other tool-engagement feature so that a tool such as a wrench, nut driver or screwdriver may be employed to tighten the assembly bolt into threaded bore 48 in the distal end of the stem. The head 88 of adapter bolt 82 may also be provided with a hex-head, locking nut or other tool-engagement feature so that a tool such as a wrench, nut driver or screwdriver may be employed to tighten the adapter bolt into the threaded bore in the distal end 78 of the percutaneous post.

The implant assembly described herein can be used in a method of securing an exo-prosthesis to a selected bone of a limb of a subject. It is contemplated that a medullary cavity of the selected bone is surgically prepared for receipt of the implant assembly. The stem, subdermal component and percutaneous post can be assembled together by means of the assembly bolt so that the stem may be positioned within the prepared site of the selected bone so that a surface defined by at least one of the distal end of the stem and the proximal portion of the subdermal component engages an osteotomy created by a cut through the long bone that is generally perpendicular to the longitudinal axis of the long bone.

Upon secure receipt of the stem within the prepared site such that the longitudinal axis of the stem is substantially axially aligned with the longitudinal axis of the selected bone, the stem can be configured to promote integration of the selected bone of the subject and the subdermal component can be configured to promote soft tissue fixation of the subject into the assembly. More particularly, it is contemplated that the structures of these components can promote integration of bone and soft tissue of the subject into the assembly such that a seal is formed between the soft tissue of the subject and the implant assembly. It is further contemplated that the seal that is formed between the soft tissue of the subject and the implant assembly can reduce the likelihood of infection in the subject and minimize the possibility of an open path of access from the exterior to the osteotomy. The optional stoma shield and adapter can then be attached to the implant assembly to allow for secure connection to an exo-prosthesis by means such as are known to those having ordinary skill in the art to which the invention relates.

It is contemplated that one or more of the individual components of the implant assembly described herein can be provided in the form of a kit. It is further contemplated that the respective components of the implant assembly described herein can be labeled or color-coded to indicate the particular sizing and/or attachment features of the component that enable a surgeon or other medical practitioner to determine whether the component is appropriate use in a particular procedure and/or whether the component is complementary in size and/or function to other components of an implant assembly.

Although this description contains many specifics, these should not be construed as limiting the scope of the invention according to the appended claims but as merely providing illustrations of the presently preferred embodiments thereof, as well as the best mode contemplated by the inventors of carrying out the invention. The invention, as described herein, is susceptible to various modifications and adaptations, as would be understood by those having ordinary skill in the art to which the invention relates, and the same are intended to be comprehended within the
scope of the invention as defined by the following claims.

## Claims

1. An implant assembly for a long bone having a longitudinal axis, which assembly is adapted to support an exo-prosthesis, said assembly comprising:
(a) a stem (22, 122, 222) having:
(i) a proximal end (34) that is adapted to be received in a surgically prepared medullary canal of the long bone;
(ii) a distal end (40, 140, 240) having a surface that is adapted to promote bone ingrowth;
(b) a subdermal component (24, 124, 224, 324) having:
(i) a proximal portion (44, 153, 262, 362) that is adapted for attachment to the distal end (40; 140;240) of the stem (22, 122, 222), wherein the proximal portion comprises a fixation surface (55, 155, 260, 360) that is adapted to promote soft tissue fixation;
(ii) a distal portion (60,160) having a low energy surface that is adapted to inhibit bioadhesion; and
(c) a percutaneous post (26) having:
(i) a proximal end (68) that is adapted to be attached to the subdermal component (24; 124;224;324);
(ii) a distal portion (76) having a distal end (78) that is threaded to receive a threaded end (80) of an adapter bolt (82),
wherein:
the percutaneous post (26) includes a low energy surface that is adapted to inhibit bioadhesion; and
at least one of the distal end (40, 140, 240) of the stem (22, 122, 222) and the proximal portion (44) of the subdermal component (24, 124, 224, 324) defines a surface (54, 154, 254, 354) that is adapted to engage an osteotomy created by a cut through the long bone that is generally perpendicular to the longitudinal axis of the long bone;
**characterised in that**:
the subdermal component (24, 124, 224, 324) further comprises:
(iii) an intermediate portion (62,162) between the distal portion (60, 160) and the proximal portion (53, 153), said intermediate portion (62, 162) having a transition surface between the fixation surface (55, 155) and the low energy surface, the transition surface comprising a surface that has been treated to increase its surface energy relative to the low energy surface yet still have a lower surface energy than the fixation surface (55, 155); and
the proximal end (68) of the percutaneous post (26) includes one or more anti-rotation tabs (75) adapted for transmission of assembly torques through the tabs and the percutaneous post (26) and away from the bone-implant interface.

2. The implant assembly of claim 1 further including an adapter (28) having:
(i) a proximal end (84);
(ii) a distal end (86) that is adapted to be attached to the exo-prosthesis;
wherein the percutaneous post (26) has a distal end (76) to which the proximal end (84) of the adapter (28) is adapted to be attached.

3. The implant assembly of claim 1 or 2, wherein the proximal portion (44) of the subdermal component (24, 124) includes a collar (53, 153) that has a radial dimension that is greater than the radial dimension of the distal end (40, 140) of the stem (22, 122), which proximal portion (44) defines a collar (53, 153) that is adapted to engage an osteotomy created by a cut through the long bone that is generally perpendicular to the longitudinal axis of the long bone.

4. The implant assembly of claim 1 wherein the subdermal component (324) includes a distal ring (360) that has a radial dimension that is greater than that of the proximal portion (362) of the subdermal component (324).

5. The implant assembly of any preceding claim, wherein at least a portion of the subdermal component (24, 124, 224, 324) has a convex profile.

6. The implant assembly of any preceding claim, wherein at least a portion of the subdermal component (24, 124 224, 324) has a concave profile.

7. The implant assembly of any preceding claim, wherein the surface of the distal end (40, 140, 240) of the stem (22, 122, 222) comprises a porous structure that is adapted to support osseointegration.

8. The implant assembly of any preceding claim, wherein the low energy surface of the distal portion (60; 160) of the subdermal component (24, 124) comprises a surface that has been treated to increase its hydrophobic characteristics.

9. The implant assembly of any preceding claim, wherein the low energy surface of the distal portion (60, 160) of the subdermal component (24, 124) is obtained by a process selected from the group consisting of abrasive blasting, burnishing, grinding, buffing, chemical vapor deposition and combinations of such processes.

10. The implant assembly of any preceding claim, wherein the fixation surface (55, 155) of the proximal portion (53, 153) of the subdermal component (24, 124) is adapted to facilitate soft tissue integration.

11. The implant assembly of any preceding claim, wherein the fixation surface (55, 155) of the proximal portion (53, 153) of the subdermal component (24, 124) comprises a porous structure that is adapted to support osseointegration.

12. The implant assembly of any preceding claim, wherein the transition surface of the intermediate portion (62, 162) of the subdermal component (24, 124) is obtained by a process selected from the group consisting of abrasive blasting, burnishing, grinding, buffing, chemical vapor deposition and combinations of such processes.

13. The implant assembly of claim 1, further comprising:
(d) an adapter (28) having a central bore that is adapted to receive the distal end (78) of the percutaneous post (26);
(e) an assembly bolt (52) comprising:
(i) a proximal portion (50) with a threaded proximal end, said proximal portion (50) having a proximal diameter that is adapted to be received in the proximal portion of the central bore (66, 166) of the percutaneous post (26) and said threaded proximal end being adapted to engage the threaded bore (48,148) in the distal end (40, 140) of the stem (22, 122);
(ii) a distal portion (84) having a distal end and a distal diameter that is adapted to be received in the distal portion (76) of the central bore of the percutaneous post (26);
(f) an adapter bolt (82) that is threaded to engage the threaded bore in the distal end (78) of the percutaneous post (26).

## Patentansprüche

1. Implantatanordnung für einen langen Knochen mit einer Längsachse, wobei die Anordnung derart ausgestaltet ist, dass sie eine Exo-Prothese unterstützt, wobei die Anordnung umfasst:
(a) einen Schaft (22, 122, 222), der
(i) ein proximales Ende (34), das derart ausgestaltet ist, dass es in einem operativ vorbereiteten medullären Kanal des langen Knochens aufgenommen werden kann,
(ii) ein distales Ende (40, 140, 240), das eine Oberfläche aufweist, die derart ausgestaltet ist, dass sie das Einwachsen des Knochens fördert,
aufweist;
(b) eine subdermale Komponente (24, 124, 224, 324), die
(i) einen proximalen Abschnitt (44, 153, 262, 362), der zur Befestigung an das distale Ende (40, 140, 240) des Schafts (22, 122, 222) ausgestaltet ist, wobei der proximale Anteil eine Befestigungsoberfläche (55, 155, 260, 360) umfasst, die zur die Förderung der Befestigung von weichem Gewebe ausgestaltet ist,
(ii) einen distalen Abschnitt (60, 160) mit einer Niedrigenergie-Oberfläche, die derart ausgestaltet ist, dass sie Bio-Adhäsion verhindert,
aufweist
und
(c) einen perkutanen Pflock (26), der
(i) ein proximales Ende (68), das derart ausgestaltet ist, dass es an der subdermalen Komponente (24, 124, 224, 324) befestigt werden kann,
(ii) einen distalen Abschnitt (76), der ein distales Ende (78) aufweist, das mit einem Gewinde versehen ist, so dass es ein mit einem Gewinde versehenes Ende (80) eines Adapterbolzens (82) aufnehmen kann,
aufweist, wobei
der perkutane Pflock (26) eine Niedrigenergie-Oberfläche aufweist, die derart ausgestaltet ist, dass sie Bio-Adhäsion verhindert und
mindestens eines aus dem distalen Ende (40, 140, 240) des Schafts (22, 122, 222) und dem proximalen Abschnitt (44) der subdermalen Komponente (24, 124, 224, 324) eine Oberfläche (54, 154, 254, 354) bildet, die derart ausgestaltet ist, dass sie in eine Knochendurchtrennung eingreift, die durch einen Schnitt durch den langen Knochen erfolgt und insgesamt senkrecht zur Längsachse des langen Knochens ist;
**dadurch gekennzeichnet, dass**
die subdermale Komponente (24, 124, 224, 324) zudem
(iii) einen Zwischenabschnitt (62, 162) zwischen dem distalen Abschnitt (60, 160) und dem proximalen Abschnitt (53, 153), wobei der Zwischenabschnitt (62, 162) eine Übergangsoberfläche zwischen der Befestigungsoberfläche (55, 155) und der Niedrigenergie-Oberfläche aufweist, wobei die Übergangsoberfläche eine Oberfläche umfasst, die derart behandelt wurde, dass ihre Oberflächenenergie gegenüber der Niedrigenergie-Oberfläche erhöht ist, sie jedoch weiterhin eine niedrigere Oberflächenenergie als die Befestigungsoberfläche (55, 155) hat
umfasst, und
das proximale Ende (68) des perkutanen Pflocks (26) ein oder mehrere Antirotationselemente (75) beinhaltet, die zur Übertragung von Drehmomenten der Anordnung durch die Elemente und den perkutanen Pflock (26) und weg von der Knochen-Implantat-Grenzfläche ausgestaltet sind.

2. Implantatanordnung gemäß Anspruch 1, zudem umfassend:
einen Adapter (28), der
(i) ein proximales Ende (84)
(ii) ein distales Ende (86), das zur Befestigung an die Exo-Prothese ausgestaltet ist,
aufweist,
und wobei der perkutane Pflock (26) ein distales Ende (76) aufweist, an welches das proximale Ende (84) des Adapters (28) zur Befestigung ausgestaltet ist.

3. Implantatanordnung gemäß Anspruch 1 oder 2, wobei der proximale Abschnitt (44) der subdermalen Komponente (24, 124) einen Kragen (53, 153) beinhaltet, dessen radiales Ausmaß größer ist als das des distalen Endes (40, 140) des Schafts (22, 122) und wobei der proximale Abschnitt (44) einen Kragen (53, 153) bildet, der derart ausgestaltet ist, dass er in eine Knochendurchtrennung eingreift, die durch einen Schnitt durch den langen Knochen erfolgt und insgesamt senkrecht zur Längsachse des langen Knochens ist.

4. Implantatanordnung gemäß Anspruch 1, wobei die subdermale Komponente (324) einen distalen Ring (360) beinhaltet, dessen radiales Ausmaß größer ist als das des proximalen Abschnittes (362) der subdermalen Komponente (324).

5. Implantatanordnung gemäß einem der vorangehenden Ansprüche, wobei mindestens ein Abschnitt der subdermalen Komponente (24, 124, 224, 324) ein konvexes Profil aufweist.

6. Implantatanordnung gemäß einem der vorangehenden Ansprüche, wobei mindestens ein Abschnitt der subdermalen Komponente (24, 124, 224, 324) ein konkaves Profil aufweist.

7. Implantatanordnung gemäß einem der vorangehenden Ansprüche, wobei die Oberfläche des distalen Endes (40, 140, 240) des Schafts (22, 122, 222) eine poröse Struktur umfasst, die zur Unterstützung der Knochenintegration ausgestaltet ist.

8. Implantatanordnung gemäß einem der vorangehenden Ansprüche, wobei die Niedrigenergie-Oberfläche des distalen Abschnitts (60, 160) der subdermalen Komponente (24, 124) eine Oberfläche umfasst, die derart behandelt wurde, dass ihre hydrophoben Eigenschaften gesteigert wurden.

9. Implantatanordnung gemäß einem der vorangehenden Ansprüche, wobei die Niedrigenergie-Oberfläche des distalen Abschnitts (60, 160) der subdermalen Komponente (24, 124) erhalten wird durch ein Verfahren ausgewählt aus der Gruppe bestehend aus Strahlen, Brünieren, Schleifen, Abrauen, chemischer Aufdampfung und Kombinationen solcher Verfahren.

10. Implantatanordnung gemäß einem der vorangehenden Ansprüche, wobei die Befestigungsoberfläche (55, 155) des proximalen Abschnitts (53, 153) der subdermalen Komponente (24, 124) zur Erleichterung der Integration von weichem Gewebe ausgestaltet ist.

11. Implantatanordnung gemäß einem der vorangehenden Ansprüche, wobei die Befestigungsoberfläche (55, 155) des proximalen Abschnitts (53, 153) der subdermalen Komponente (24, 124) eine poröse Struktur umfasst, die zur Unterstützung der Knochenintegration ausgestaltet ist.

12. Implantatanordnung gemäß einem der vorangehenden Ansprüche, wobei die Übergangsoberfläche des Zwischenabschnitts (62, 162) der subdermalen Komponente (24, 124) erhalten wird durch ein Verfahren ausgewählt aus der Gruppe bestehend aus Strahlen, Brünieren, Schleifen, Abrauen, chemischer Aufdampfung und Kombinationen solcher Verfahren.

13. Implantatanordnung gemäß Anspruch 1, zudem umfassend:
(d) einen Adapter (28), der eine zentrale Bohrung aufweist, die zur Aufnahme des distalen Endes (78) des perkutanen Pflocks (26) ausgestaltet ist,
(e) einen Montagebolzen (52) umfassend:
(i) einen proximalen Abschnitt (50) mit einem mit einem Gewinde versehenen proximalen Ende, wobei der proximale Abschnitt (50) einen proximalen Durchmesser aufweist, der derart ausgestaltet ist, dass er im proximalen Abschnitt der zentralen Bohrung (66, 166) des perkutanen Pflocks (26) aufgenommen werden kann und wobei das mit einem Gewinde versehene Ende derart ausgestaltet ist, dass es mit der mit einem Gewinde versehenen Bohrung (48, 148) im distalen Ende (40, 140) des Schafts (22, 122) ineinandergreift,
(ii) einen distalen Abschnitt (84), der ein distales Ende und einen distalen Durchmesser aufweist, der ausgestaltet ist, um in dem distalen Abschnitt (76) der zentralen Bohrung des perkutanen Pflocks (26) aufgenommen zu werden,
(f) einen Adapterbolzen (82), der mit einem Gewinde versehen ist, um mit der mit einem Gewinde versehenen Bohrung im distalen Ende (78) des perkutanen Pflocks (26) ineinanderzugreifen.

## Revendications

1. Ensemble implant pour un os long ayant un axe longitudinal, lequel ensemble est adapté pour supporter une exo-prothèse, ledit ensemble comprenant :
(a) une tige (22, 122, 222) ayant :
(i) une extrémité proximale (34) qui est adaptée pour être reçue dans un canal médullaire préparé chirurgicalement de l'os long ;
(ii) une extrémité distale (40, 140, 240) ayant une surface qui est adaptée pour favoriser la croissance osseuse interne ;
(b) un composant sous-cutané (24, 124, 224, 324) ayant:
(i) une partie proximale (44, 153, 262, 362) qui est adaptée pour être attachée à l'extrémité distale (40; 140 ; 240) de la tige (22, 122, 222), où la partie proximale comprend une surface de fixation (55, 155, 260, 360) qui est adaptée pour favoriser la fixation de tissus mous;
(ii) une partie distale (60, 160) ayant une surface de faible énergie qui est adaptée pour inhiber la bioadhésion ; et
(c) un tenon percutané (26) ayant :
(i) une extrémité proximale (68) qui est adaptée pour être attachée au composant sous-cutané (24 ; 124 ; 224 ; 324) ;
(ii) une partie distale (76) ayant une extrémité distale (78) qui est filetée pour recevoir une extrémité filetée (80) d'un boulon d'adaptateur (82),
dans lequel :
le tenon percutané (26) comporte une surface de faible énergie qui est adaptée pour inhiber la bioadhésion ; et
au moins l'une de l'extrémité distale (40, 140, 240) de la tige (22, 122, 222) et de la partie proximale (44) du composant sous-cutané (24, 124, 224, 324) définit une surface (54, 154, 254, 354) qui est adaptée pour s'engager avec une ostéotomie créée par une coupe à travers l'os long qui est généralement perpendiculaire à l'axe longitudinal de l'os long ;
**caractérisé en ce que** :
le composant sous-cutané (24, 124, 224, 324) comprend en outre :
(iii) une partie intermédiaire (62, 162) entre la partie distale (60, 160) et la partie proximale (53, 153), ladite partie intermédiaire (62, 162) ayant une surface de transition entre la surface de fixation (55, 155) et la surface de faible énergie, la surface de transition comprenant une surface qui a été traitée pour augmenter son énergie de surface par rapport à la surface de faible énergie tout en ayant une énergie de surface inférieure à celle de la surface de fixation (55, 155) ; et
l'extrémité proximale (68) du tenon percutané (26) comporte une ou plusieurs languette(s) anti-rotation (75) adaptée(s) pour la transmission de couples d'assemblage à travers les languettes et le tenon percutané (26) et loin de l'interface os-implant.

2. Ensemble implant de la revendication 1 comportant en outre un adaptateur (28) ayant :
(i) une extrémité proximale (84) ;
(ii) une extrémité distale (86) qui est adaptée pour être attachée à l'exo-prothèse ;
dans lequel le tenon percutané (26) a une extrémité distale (76) à laquelle l'extrémité proximale (84) de l'adaptateur (28) est adaptée pour être attachée.

3. Ensemble implant de la revendication 1 ou 2, dans lequel la partie proximale (44) du composant sous-cutané (24, 124) comporte un collier (53, 153) qui a une dimension radiale qui est supérieure à la dimension radiale de l'extrémité distale (40, 140) de la tige (22, 122), laquelle partie proximale (44) définit un collier (53, 153) qui est adapté pour s'engager avec une ostéotomie créée par une coupe à travers l'os long qui est généralement perpendiculaire à l'axe longitudinal de l'os long.

4. Ensemble implant de la revendication 1, dans lequel le composant sous-cutané (324) comporte une bague distale (360) qui a une dimension radiale qui est supérieure à celle de la partie proximale (362) du composant sous-cutané (324).

5. Ensemble implant de l'une des revendications précédentes, dans lequel au moins une partie du composant sous-cutané (24, 124, 224, 324) a un profil convexe.

6. Ensemble implant de l'une des revendications précédentes, dans lequel au moins une partie du composant sous-cutané (24, 124, 224, 324) a un profil concave.

7. Ensemble implant de l'une des revendications précédentes, dans lequel la surface de l'extrémité distale (40, 140, 240) de la tige (22, 122, 222) comprend une structure poreuse qui est adaptée pour supporter l'ostéo-intégration.

8. Ensemble implant de l'une des revendications précédentes, dans lequel la surface de faible énergie de la partie distale (60 ; 160) du composant sous-cutané (24, 124) comprend une surface qui a été traitée pour augmenter ses caractéristiques hydrophobes.

9. Ensemble implant de l'une des revendications précédentes, dans lequel la surface de faible énergie de la partie distale (60, 160) du composant sous-cutané (24, 124) est obtenue par un procédé choisi dans le groupe consistant en l'abrasion par projection, le brunissage, le meulage, le bufflage, le dépôt chimique en phase vapeur et des combinaisons de tels procédés.

10. Ensemble implant de l'une des revendications précédentes, dans lequel la surface de fixation (55, 155) de la partie proximale (53, 153) du composant sous-cutané (24, 124) est adaptée pour faciliter l'intégration de tissus mous.

11. Ensemble implant de l'une des revendications précédentes, dans lequel la surface de fixation (55, 155) de la partie proximale (53, 153) du composant sous-cutané (24, 124) comprend une structure poreuse qui est adaptée pour supporter l'ostéo-intégration.

12. Ensemble implant de l'une des revendications précédentes, dans lequel la surface de transition de la partie intermédiaire (62, 162) du composant sous-cutané (24, 124) est obtenue par un procédé choisi dans le groupe consistant en l'abrasion par projection, le brunissage, le meulage, le bufflage, le dépôt chimique en phase vapeur et des combinaisons de tels procédés.

13. Ensemble implant de la revendication 1, comprenant en outre :
(d) un adaptateur (28) ayant un alésage central qui est adapté pour recevoir l'extrémité distale (78) du tenon percutané (26) ;
(e) un boulon d'assemblage (52) comprenant :
(i) une partie proximale (50) avec une extrémité proximale filetée, ladite partie proximale (50) ayant un diamètre proximal qui est adapté pour être reçu dans la partie proximale de l'alésage central (66, 166) du tenon percutané (26) et ladite extrémité proximale filetée étant adaptée pour engager l'alésage fileté (48, 148) dans l'extrémité distale (40, 140) de la tige (22, 122) ;
(ii) une partie distale (84) ayant une extrémité distale et un diamètre distal qui est adapté pour être reçu dans la partie distale (76) de l'alésage central du tenon percutané (26) ;
(f) un boulon d'adaptateur (82) qui est fileté pour engager l'alésage fileté dans l'extrémité distale (78) du tenon percutané (26).
